# EUROPEAN PATENT APPLICATION

(11) **EP 2 848 137 A1**
(43) Date of publication of application: **18.03.2015**
(21) Application number: 14184879.6
(22) Date of filing: 16.09.2014
(51) Int. Cl.: A41D 19/015, A61B 17/072

(54) **Surgical instrument controls with illuminated feedback**

(30) Priority: 17.09.2013 US 201361878916 P; 08.08.2014 US 201414454966
(71) Applicant: Covidien LP, Mansfield, MA 02048 (US)
(72) Inventor: Irka, Philip Joseph, Northford, CT Connecticut 06472 (US); Chowaniec, Matthew J., Middletown, CT Connecticut 06457 (US); Ingmanson, Michael D., Stratford, CT Connecticut 06614 (US)
(74) Representative: Soames, Candida Jane

(57) **Abstract**

An electromechanical powered surgical instrument (100) includes a handle assembly (102) and a control assembly (400). The handle assembly is configured to engage an end effector (300) to manipulate a tool assembly (304) thereof. The control assembly (400) is positioned on the handle assembly and includes a plurality of buttons (410-418). Each of the plurality of buttons (410-418) is associated with a function of the end effector. Each of the plurality of buttons (410-418) is also associated with a light source. The light source is configured to selectively backlight the associated button.

## Description

### CROSS-REFERENCE TO RELATED APPLICATIONS

This application claims the benefit of and priority to U.S. Provisional Patent Application No. 61/878,916, filed September 17, 2013, the entire disclosure of which is incorporated by reference herein.

### 1. Technical Field

The present disclosure relates to surgical instruments and, more specifically, to surgical instruments controls that provide illuminated feedback.

### 2. Discussion of Related Art

Traditional methods of communicating information, i.e., providing feedback, to clinicians include display screens, markings, and small indication lights. These methods require clinicians to focus on small and specific areas of an instrument. This can distract the clinicians from other areas and can be difficult for the clinicians to interpret once the clinician's full attention is devoted to the specific area of the instrument.

There is a continuing need to provide surgical instruments with improved feedback systems that improve the clinician's ability to learn the instrument and focus on the patient during surgery.

### SUMMARY

In aspects of the present disclosure, a powered surgical instrument for operating an end effector includes a handle assembly and a control assembly. The handle assembly is configured to engage the end effector. The control assembly is positioned on the handle assembly and includes a plurality of buttons. Each of the plurality of buttons is associated with a function of the end effector and is associated with a light source. Each light source is configured to selectively backlight the associated button. Each light source may be configured to selectively backlight the associated button with diffused light. In some embodiments, each of the plurality of buttons is configured to selectively backlight the associated button with sufficient light to diffuse through a semi-opaque object when the object is disposed over the backlit button. In embodiments, each of the buttons is backlit when the function associated with the button is activatable and is not backlit the function associated with the button is not activatable.

In aspects of the present disclosure, two of the buttons are backlit with a respective intensity of light. A first of the buttons may be backlit with a first intensity of light and a second of the buttons may be backlit with a second intensity of light. The second intensity of light being different from the first intensity of light. The intensity of light being based on the function associated with each of the two buttons.

In aspects of the present disclosure, two of the buttons are backlit with a respective color of light. A first of the two buttons may be backlit with a first color of light and a second of the two of the buttons may be backlit with a second color of light. The second color of light being different from the first color of light. The color of light being based on the function associated with each of the two buttons.

In aspects of the present disclosure, two of the buttons are backlit with light flashing in a respective sequence. A first of the two buttons may be backlit with light flashing in a first sequence and a second of the two buttons may be backlit with light flashing in a second sequence. The second sequence being different from the first sequence. The sequence being based on the function associated with each of the two buttons.

In aspects of the present disclosure, one of the buttons is backlit with a first color when a first function is associated with the button and is backlit with a second color when a second function is associated with the button.

In aspects of the present disclosure, the control assembly includes a light sensor electrically associated with the light sources. The light sensor is configured to sense a lighting condition of the environment surrounding the control assembly and is configured to adjust the intensity of the light source in response to the lighting condition. The light sensor may be a photovoltaic sensor.

In aspects of the present disclosure, one of the light sources is configured to diffuse light about a surface of a glove adjacent the button associated with the light source. In embodiments, the light source is configured to diffuse light through a glove adjacent the button associated with the light source. Each light source may be configured to change a characteristic of the diffused light in response to a distance the glove is away from the button associated with the respective light source. The button associated with each light source may include a cell configured to sense the distance the glove is away from the button associated with the respective light source. Each light source may pulse the diffused light in a first sequence when the glove is at a first distance from the associated button and pulse the diffused light in a second sequence when the glove is at a second distance from the associated button.

In aspects of the present disclosure, one of the buttons includes a first icon as indicia of a first function that is associated with the button and a second icon as indicia of a second function that is associated with the button. The first icon being viewable when the first function is associated with the button and the second icon being viewable when the second function is associated with the button. In embodiments, the first and second icons are not simultaneously viewable.

In aspects of the present disclosure, the control assembly includes a touch interface that selectively defines each of the buttons when the function associated with the button is activatable.

In aspects of the present disclosure, an electromechanical surgical system includes an end effector and a powered surgical instrument. The end effector includes a tool assembly having a cartridge assembly and an anvil assembly. The cartridge assembly and the anvil assembly are moveable relative to one another between an open position and a closed position. In the open position, the cartridge assembly is spaced-spaced apart from the anvil assembly and in the closed position, the cartridge assembly is approximated to the anvil assembly.

Further, to the extent consistent, any of the aspects described herein may be used in conjunction with any or all of the other aspects described herein.

### BRIEF DESCRIPTION OF THE DRAWINGS

Various aspects of the present disclosure are described hereinbelow with reference to the drawings, wherein:
FIG. 1 is a rear perspective view of an exemplary embodiment of an electromechanical, hand-held, powered surgical system in accordance with the present disclosure including a surgical instrument having a tool assembly in an open position and a control assembly with backlit buttons;
FIG. 2 is a rear view of the surgical system of FIG. 1;
FIG. 3 is a rear perspective view of the surgical system of FIG. 1 with tool assembly in a closed position and a finger of a clinician engaging the close/fire button;
FIG. 3A is a schematic side-section view taken along line 3A-3A shown in FIG. 3;
FIG. 4 is a rear perspective view of another exemplary embodiment of an electromechanical, hand-held, powered surgical system in accordance with the present disclosure including a surgical instrument having a tool assembly in an open position and a control assembly including a touch screen; and
FIG. 5 is a rear perspective view of the surgical system of FIG. 4 with the tool assembly in a closed position and a finger of a clinician engaging the fire button.

### DETAILED DESCRIPTION

Embodiments of the presently disclosed electromechanical surgical system, apparatus, and/or device are described in detail with reference to the drawings, in which like reference numerals designate identical or corresponding elements in each of the several views. As used herein the term "distal" refers to that portion of the electromechanical surgical system, apparatus and/or device, or component thereof, that are farther from the user, while the term "proximal" refers to that portion of the electromechanical surgical system, apparatus and/or device, or component thereof, that are closer to the user. The terms "left" and "right" refer to that portion of the electromechanical surgical system, apparatus, device, and/or component thereof, that are on the left and right sides, respectively, from the perspective of the user facing the distal end of the electromechanical surgical system, apparatus and/or instrument from the proximal end while the surgical system, apparatus and/or device is oriented in non-rotational (e.g., home) configuration. The term "clinician" refers to a doctor, a nurse, or any other care provider and may include support personnel.

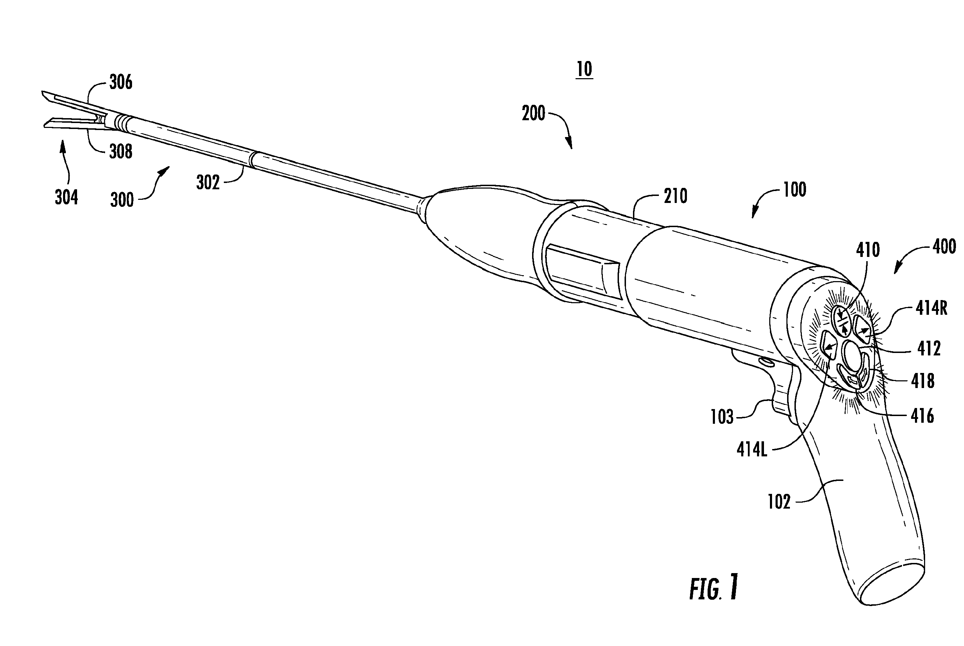

Referring initially to FIG. 1, an electromechanical, hand-held, powered surgical system, in accordance with an embodiment of the present disclosure is shown and generally designated 10. Electromechanical surgical system 10 includes a surgical apparatus or device in the form of an electromechanical, hand-held, powered surgical instrument 100 that is configured for selective attachment thereto of a plurality of different end effectors 300, via an adapter assembly 200 (e.g., elongated body). End effector 300 and adapter assembly 200 are configured for actuation and manipulation by the electromechanical, hand-held, powered surgical instrument 100. In particular, surgical instrument 100, adapter assembly 200, and end effector 300 are separable from each other such that surgical instrument 100 is configured for selective connection with adapter assembly 200, and, in turn, adapter assembly 200 is configured for selective connection with any one of a plurality of different end effectors 300.

A brief description of the construction an operation of surgical instrument 100 is included herein. For a detailed description of the construction and operation of exemplary electromechanical, hand-held, powered surgical instrument 100 reference may be made to International Application No. PCT/US2008/077249, filed September 22, 2008 (Inter. Pub. No. WO 2009/039506), U.S. Patent Pub. No. 2011/0121049, and U.S. Provisional Patent Application Serial No. 61/837,225 [H-US-03825 (203-9270)], the entire contents of each of which is incorporated herein by reference.

Surgical instrument 100 may include a handle housing 102 including one or more controllers (not shown), a power source (not shown), and a drive mechanism (not shown) having one or more motors, gear selector boxes, gearing mechanisms, and the like disposed therein.

In particular, the drive mechanism may be configured to, for example, drive shafts and/or gear components (not shown) in order to selectively articulate a tool assembly 304 of end effector 300 relative to a proximal body portion 302 of end effector 300, to rotate end effector 300 about a longitudinal axis thereof, to move anvil assembly 306 and cartridge assembly 308 of end effector 300 relative to one another, to fire staples from cartridge assembly 308, and/or advancing a knife within cartridge assembly 308.

Continuing to refer to FIG. 1, housing 102 supports a control assembly 400 whose input is transferred to the drive mechanism of surgical instrument 100 to manipulate end effector 300 and tool assembly 304 thereof as detailed above. As shown, control assembly 400 includes a close/fire button 410, an unclamp or open button 412, left and right articulation buttons 414L, 414R, a clockwise rotational button 416, and a counter-clockwise rotational button 418. It is contemplated that control assembly 400 may include a one or more finger-actuated control buttons, rocker devices, touch screens, joystick, or other directional controls.

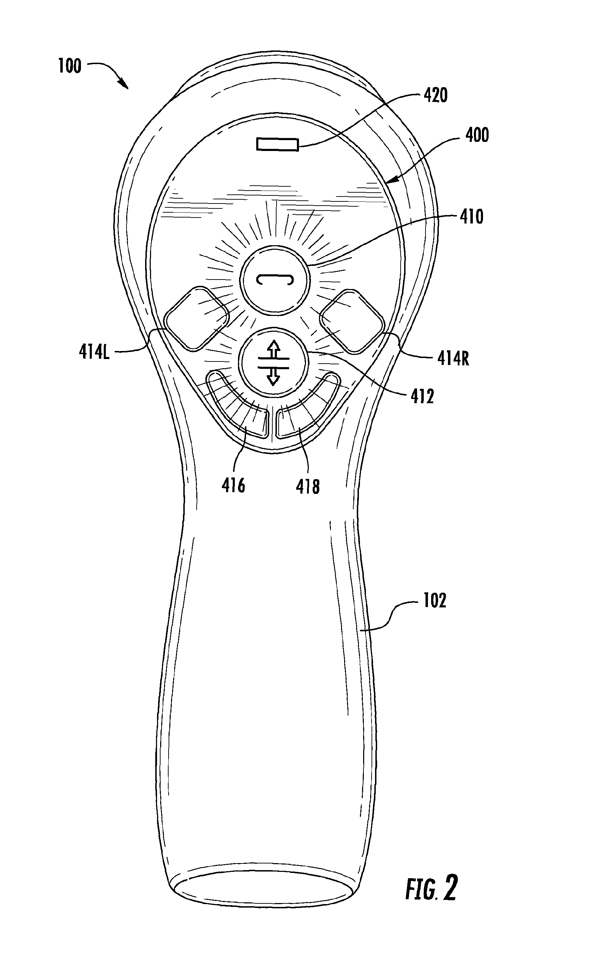

Each button 410-418 may include indicia indicative of the function of the button 410-418 inscribed or projected on the surface thereof. The indicia may change or disappear as an operative condition of components of surgical system 10 changes, e.g., in FIG. 1 button 410 displays a first icon indicating the close function and in FIG. 2 button 410 displays a second icon indicating the fire function.

For example, buttons 410-418 may be selectively backlit as detailed below. The material and/or surface of each of buttons 410-418 is sufficiently clear or translucent so as to diffuse the light that backlights the button 410-418. In embodiments, the material and/or surface of each of buttons 410-418 is sufficiently clear or translucent so as to diffuse the light that backlights the button 410-418 through an object placed over the button, such as, for example, a user's gloved finger. An example of materials that may be used for buttons 410-418 are translucent polymers. The diffusion of the light by each button 410-418 may increase the visibility of buttons 410-418 as compared to a backlit button that does not diffuse light. Each button 410-418 may be backlit in a color to provide indicia of the function of the button 410-418. Each button 410-418 may be backlit in a color different from each other button 410-418. Each button 410-418 may include a single backlighting element, i.e., an LED light, configured to backlight the button 410-418 in a single color or multiple backlighting elements, i.e., multiple LED lights of the same or differing colors configured to backlight the button 410-418 in multiple colors based on the current function of the button 410-418. Differing the color of buttons 410-418 provides feedback to a clinician, which may assist the clinician in identifying the function of each button as the operative condition of the components of surgical system 10 change, e.g., as tool assembly 304 closes. In embodiments, buttons 410-418 are selectively backlit in response to sensed conditions of surgical instrument 100, end effector 300, or components thereof to only backlight buttons 410-418 corresponding to available functions.

As shown in FIG. 1, tool assembly 304 of end effector 300 is in an open position. When tool assembly 304 is in this condition a clinician may choose the articulate, rotate, or close functions of tool assembly 304 of end effector 300. As such only close/fire button 410, articulation buttons 414L, 414R, and rotational buttons 416, 418 are backlit. It will be appreciated, that while tool assembly 304 is in the open position open button 412 corresponding to opening function of tool assembly 304 is not available and is not backlit.

In this open condition of tool assembly 304, close/fire button 410 may be backlit in green and articulation buttons 414L, 414R may be backlit in yellow and rotational buttons 416, 418 may be backlit in blue to provide simplified feedback to a clinician as to the function of each button.

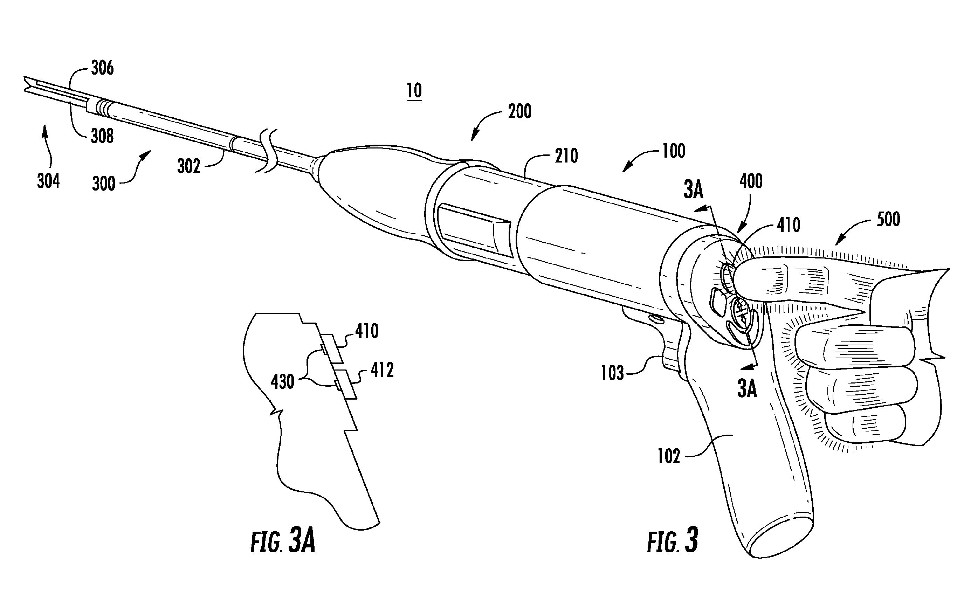

As shown in FIG. 2, the rear of instrument 100 is shown when the tool assembly (FIG. 3) is in the closed position. When tool assembly 304 is in the closed position, instrument 100 may be functionally limited to firing staples from the staple cartridge (not shown), advancing a knife within the staple cartridge, or opening the tool assembly (FIG. 3). As such only close/fire button 410 (for firing the staples or advancing the knife) and the open button 412 are backlit. In this condition, close/fire button 410 may be backlit in green and open button 412 may be backlit in red. It will further be appreciated, that as shown in FIGS. 2 and 3, while tool assembly 304 is in the closed position articulation buttons 414L, 414R and rotational buttons 416, 418 corresponding to the articulation and rotation functions of tool assembly 304, respectively, are not available and are not backlit. In some embodiments, while tool assembly 304 is in the closed position articulation buttons 414L, 414R and rotational buttons 416, 418 may be activatable and are backlit.

In embodiments, the backlighting of buttons 410-418 provides indicia and/or feedback of the functionality of each button 410-418 by flashing in different sequences and/or at different frequencies. In some embodiments, the backlighting of buttons 410-418 provides indicia of the functionality of each button 410-418 by varying the intensity of the backlighting.

Control assembly 400 may include a sensor 420 to detect a lighting condition of the environment surrounding surgical instrument 100. By detecting the lighting condition control assembly 400 may automatically vary the intensity of the backlighting of buttons 410-418 in response to the lighting condition, e.g., when in a device preparation area in a darker area of a room the intensity of the backlighting may be low and in the brightly lit surgical area of an operating room the intensity of the backlighting may be high, to improve the visuality of the buttons 410-418. Sensor 420 may be a photovoltaic sensor. In response to sensor 420, control assembly 400 may adjust the current driving the backlighting of buttons 410-418 to adjust the intensity of the backlighting.

Referring to FIG. 3, buttons 410-418 of control assembly 400 may be clear or translucent and configured to diffuse the backlighting about the surface of a glove 500 worn by a clinician or to transmit the backlighting through glove 500. Glove 500 may be translucent or semi-opaque such that light from buttons 410-418 is diffused about the outer surface of glove 500 to provide indicia that a finger or a hand of a clinician is adjacent to or in contact with a selected button of control assembly 400. The diffused light may change color when the finger contacts a select button 410-418 and may change color when the finger depresses or activates a select button 410-418. In some embodiments, the diffused light changes patterns as the finger of the clinician approaches and contacts the selected button 410-418, e.g., the diffused light increases the frequency of flashing as the finger approaches the selected button 410-418 and is solid when the finger is in contact with the selected button 410-418 and may go out when the finger depresses or activates a select button 410-418. In certain embodiments, the selected button 410-418 may include a sensor to detect the position of the finger of the clinician relative to the selected button 410-418 operatively associated with control assembly 400 to change the pattern of the diffused light. The increased surface area of diffused light may assist a clinician in selecting a desired function of surgical instrument 100 without redirecting the attention of the clinician from another area, i.e., a surgical site.

Referring to FIG. 3A, button 410 includes a cell 430, e.g., a photoelectric cell, a capacitive touch cell, a force feedback cell, etc., positioned under button 410 to monitor the level of light that enters the device to determine when the finger of the clinician is in contact with or adjacent to button 410. It is contemplated that each button 410-418 may include a cell 430.

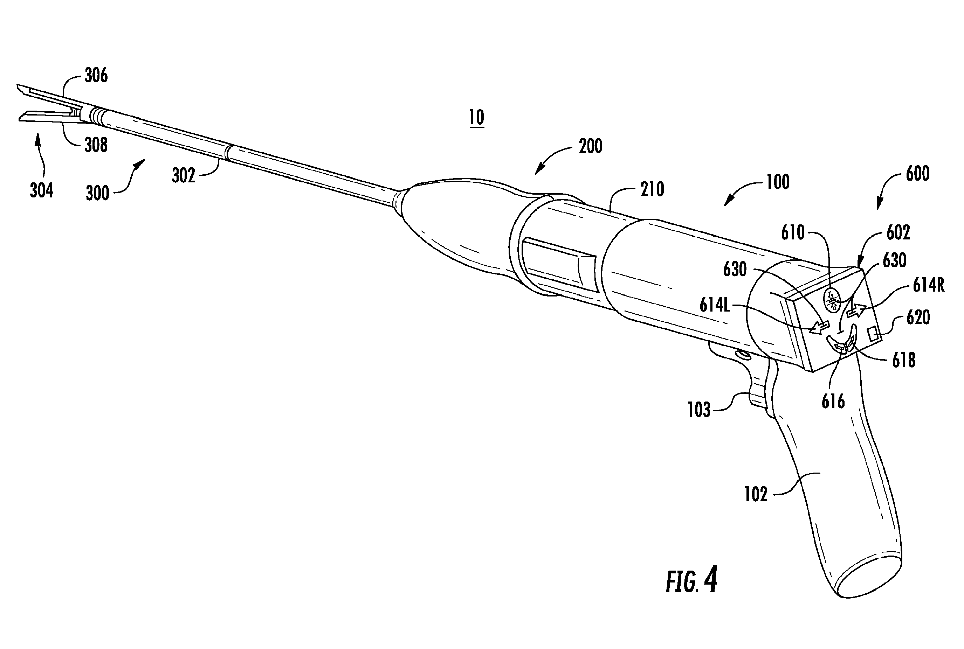

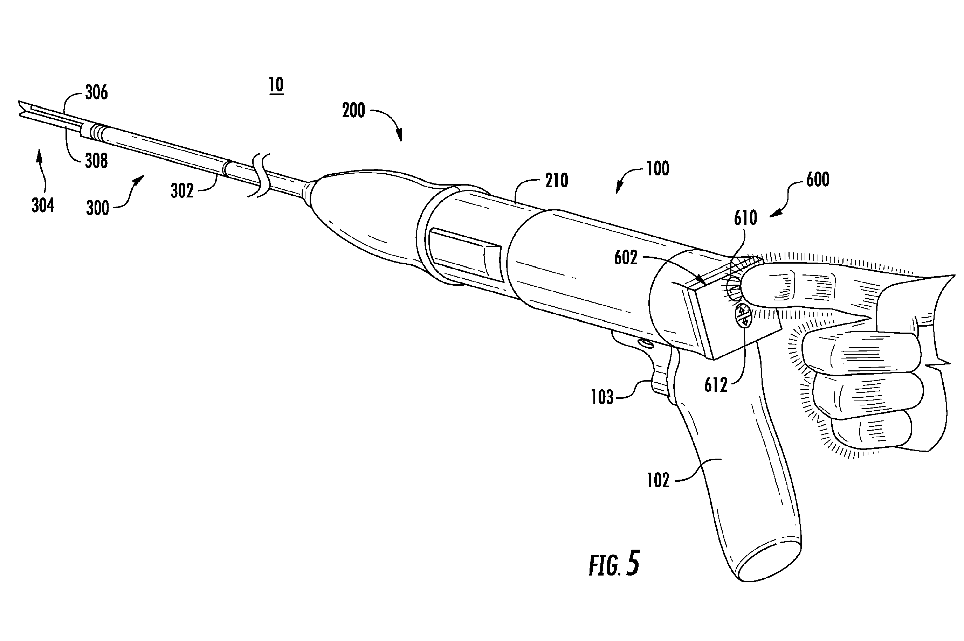

Referring to FIGS. 4 and 5, surgical instrument 100 includes a control assembly 600. Control assembly 600 is similar to control assembly 400 detailed above and only the differences will be detailed below for reasons of brevity.

Control assembly 600 includes a touch interface 602. Touch interface 602 is dynamically configurable without fixed buttons for each function of surgical instrument 100. As shown in FIG. 4, tool assembly 304 of end effector 300 is in the open position. When tool assembly 304 is in the open position, a clinician may choose the articulate, rotate, or close functions of tool assembly 304 of end effector 300. When tool assembly 304 is in such a position, touch interface 602 only displays buttons or defines areas corresponding with each function, i.e., a close/fire area 610, articulation areas 614L, 614R, and rotational areas 616, 618.

As shown in FIG. 5, tool assembly 304 of end effector 300 is in the closed position. When tool assembly 304 is in the closed position, instrument 100 may be functionally limited to firing staples from staple cartridge 308, advancing a knife within staple cartridge 308, or opening tool assembly 304. As such only close/fire area 410 (for firing the staples or advancing the knife) and an open area 412 are displayed or defined on touch interface 602.

Each area 610-618 may include indicia of its function in the form of color, iconic indicia, and/or shape. Touch interface 602 may be backlit with diffused light to selectively illuminate each area 610-618.

Control assembly 600 may include a sensor 620 to detect the lighting condition of the area surrounding control assembly 600 to vary the intensity of the backlighting of each area 610-618. Sensor 620 of control assembly 600 is similar to sensor 420 of control assembly 400. It is contemplated that each area of control assembly 600 may include a cell 630 to detect the finger of a clinician adjacent or in contact with each area. Cells 630 of control assembly 600 are similar to cells 430 of control assembly 400. Each area 610-618 may diffuse light about the outer surface of glove 500 as the finger of the clinician approaches and/or contacts a selected area 610-618. The intensity and/or frequency of the backlighting of areas 610-618 may also vary as the finger of the clinician approaches, contacts, and/or activates a selected area 610-618.

In accordance with the present disclosure, in addition to the feedback provided to the clinician by the backlighting of buttons 410-418 or the lighting of areas 610-618, the feedback to the clinician may be augmented by some haptic feedback wherein surgical instrument 100 may vibrate in response to contact or activation thereof. An intensity of the haptic feedback may vary in response to a number of conditions, including and not limited to a thickness of gloves worn by the clinician, a stage of the surgical procedure, etc.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A powered surgical instrument for operating an end effector, the powered surgical instrument comprising:
   a handle assembly configured to engage the end effector; and
   a control assembly positioned on the handle assembly, the control assembly including a plurality of buttons, each of the plurality of buttons associated with at least one corresponding function of the end effector, each of the plurality of buttons associated with a light source configured to selectively backlight the associated button.
2. The powered surgical instrument of paragraph 1, wherein each of the plurality of buttons is configured to selectively backlight the associated button with diffused light.
3. The powered surgical instrument of paragraph 1, wherein each of the plurality of buttons is configured to selectively backlight the associated button with light sufficient to diffuse through a semi-opaque object when the object is disposed over the backlit button.
4. The powered surgical instrument of paragraph 1, wherein each of the plurality of buttons is backlit when the associated function of the button is activatable, and each of the plurality of buttons is not backlit when the associated function of the button is nonactivatable.
5. The powered surgical instrument of paragraph 1, wherein at least two of the plurality of buttons are backlit with a respective intensity of light.
6. The powered surgical instrument of paragraph 5, wherein a first of the at least two of the plurality of buttons is backlit with a first intensity of light and a second of the at least two of the plurality of buttons is backlit with a second intensity of light different from the first intensity of light, the intensity of light based on the function associated with each of the at least two buttons.
7. The powered surgical instrument of paragraph 1, wherein at least two of the plurality of buttons are backlit with a respective color of light.
8. The powered surgical instrument of paragraph 7, wherein a first of the at least two of the plurality of buttons is backlit with a first color of light and a second of the at least two of the plurality of buttons is backlit with a second color of light different from the first color of light, the color of light based on the function associated with each of the at least two buttons.
9. The powered surgical instrument of paragraph 1, wherein at least two of the plurality of buttons are backlit with light flashing in a respective sequence.
10. The powered surgical instrument of paragraph 9, wherein a first of the at least two of the plurality of buttons is backlit with light flashing in a first sequence and a second of the at least two of the plurality of buttons is backlit with light flashing in a second sequence different from the first sequence, the sequence based on the function associated with each of the at least two buttons.
11. The powered surgical instrument of paragraph 1, wherein at least one of the plurality of buttons is backlit with a first color when a first function is associated with the at least one button, and the at least one of the plurality of buttons is backlit with a second color when a second function is associated with the at least one button.
12. The powered surgical instrument of paragraph 1, wherein the control assembly further includes a light sensor electrically associated with the light sources, the light sensor configured to sense a lighting condition of the environment surrounding the control assembly and configured to adjust the intensity of the light source in response to the lighting condition.
13. The powered surgical instrument of paragraph 1, wherein at least one light source is configured to diffuse light about a surface of a glove adjacent the button associated with the at least one light source.
14. The powered surgical instrument of paragraph 13, wherein the at least one light source is configured to change a characteristic of the diffused light in response to a distance the glove is away from the button associated with the at least one light source.
15. The powered surgical instrument of paragraph 14, wherein the button associated with the at least one light source includes a cell configured to sense the distance the glove is away from the button associated with the at least one light source.
16. The powered surgical instrument of paragraph 14, wherein the at least one light source pulses the diffused light in a first sequence when the glove is at a first distance from the button associated with the at least one light source, and pulses the diffused light in a second sequence when the glove is at a second distance from the button associated with the at least one light source.
17. The powered surgical instrument of paragraph 1, wherein the control assembly includes a touch interface, each of the plurality of buttons is selectively defined about the touch interface when the function associated with the button is activatable.
18. The powered surgical instrument of paragraph 1, wherein at least one of the plurality of buttons includes a first icon as indicia of a first function associated with the at least one of the plurality of buttons, and a second icon as indicia of a second function associated with the at least one of the plurality of buttons.
19. The powered surgical instrument of paragraph 18, wherein the first icon is viewable when the first function associated with the at least one of the plurality of buttons is activatable, and the second icon is viewable when the second function is associated with the at least one of the plurality of buttons is activatable, the first and second icons are not simultaneously viewable on the at least one of the plurality of buttons.
20. The powered surgical instrument of paragraph 1, wherein at least one light source is configured to diffuse light through a glove adjacent the button associated with the at least one light source.

## Claims

1. A powered surgical instrument for operating an end effector, the powered surgical instrument comprising:
a handle assembly configured to engage the end effector; and
a control assembly positioned on the handle assembly, the control assembly including a plurality of buttons, each of the plurality of buttons associated with at least one corresponding function of the end effector, each of the plurality of buttons associated with a light source configured to selectively backlight the associated button.

2. The powered surgical instrument of claim 1, wherein each of the plurality of buttons is configured to selectively backlight the associated button with diffused light; and/or wherein each of the plurality of buttons is configured to selectively backlight the associated button with light sufficient to diffuse through a semi-opaque object when the object is disposed over the backlit button; and/or wherein each of the plurality of buttons is backlit when the associated function of the button is activatable, and each of the plurality of buttons is not backlit when the associated function of the button is nonactivatable.

3. The powered surgical instrument of claim 1 or claim 2, wherein at least two of the plurality of buttons are backlit with a respective intensity of light; preferably wherein a first of the at least two of the plurality of buttons is backlit with a first intensity of light and a second of the at least two of the plurality of buttons is backlit with a second intensity of light different from the first intensity of light, the intensity of light based on the function associated with each of the at least two buttons.

4. The powered surgical instrument of any preceding claim, wherein at least two of the plurality of buttons are backlit with a respective color of light; preferably wherein a first of the at least two of the plurality of buttons is backlit with a first color of light and a second of the at least two of the plurality of buttons is backlit with a second color of light different from the first color of light, the color of light based on the function associated with each of the at least two buttons.

5. The powered surgical instrument of claim 1, wherein at least two of the plurality of buttons are backlit with light flashing in a respective sequence; preferably wherein a first of the at least two of the plurality of buttons is backlit with light flashing in a first sequence and a second of the at least two of the plurality of buttons is backlit with light flashing in a second sequence different from the first sequence, the sequence based on the function associated with each of the at least two buttons.

6. The powered surgical instrument of claim 1, wherein at least one of the plurality of buttons is backlit with a first color when a first function is associated with the at least one button, and the at least one of the plurality of buttons is backlit with a second color when a second function is associated with the at least one button.

7. The powered surgical instrument of any preceding claim, wherein the control assembly further includes a light sensor electrically associated with the light sources, the light sensor configured to sense a lighting condition of the environment surrounding the control assembly and configured to adjust the intensity of the light source in response to the lighting condition.

8. The powered surgical instrument of any preceding claim, wherein at least one light source is configured to diffuse light about a surface of a glove adjacent the button associated with the at least one light source.

9. The powered surgical instrument of claim 8, wherein the at least one light source is configured to change a characteristic of the diffused light in response to a distance the glove is away from the button associated with the at least one light source; preferably wherein the button associated with the at least one light source includes a cell configured to sense the distance the glove is away from the button associated with the at least one light source.

10. The powered surgical instrument of claim 9, wherein the at least one light source pulses the diffused light in a first sequence when the glove is at a first distance from the button associated with the at least one light source, and pulses the diffused light in a second sequence when the glove is at a second distance from the button associated with the at least one light source.

11. The powered surgical instrument of any preceding claim, wherein the control assembly includes a touch interface, each of the plurality of buttons is selectively defined about the touch interface when the function associated with the button is activatable.

12. The powered surgical instrument of any preceding claim, wherein at least one of the plurality of buttons includes a first icon as indicia of a first function associated with the at least one of the plurality of buttons, and a second icon as indicia of a second function associated with the at least one of the plurality of buttons.

13. The powered surgical instrument of claim 12, wherein the first icon is viewable when the first function associated with the at least one of the plurality of buttons is activatable, and the second icon is viewable when the second function is associated with the at least one of the plurality of buttons is activatable, the first and second icons are not simultaneously viewable on the at least one of the plurality of buttons.

14. The powered surgical instrument of any preceding claim, wherein at least one light source is configured to diffuse light through a glove adjacent the button associated with the at least one light source.
